Europäisches Patentamt

European Patent Office

Office européen des brevets

(11) Numéro de publication: **0 172 595**

**B1**

(12) **FASCICULE DE BREVET EUROPÉEN**

(45) Date de publication du fascicule du brevet:
**25.01.89**

(51) Int. Cl.⁴: **C 07 D 213/61, C 07 C 17/04**

(21) Numéro de dépôt: **85201297.0**

(22) Date de dépôt: **09.08.85**

(54) **Procédé pour la préparation de dérivés chlorés de composés pyridiniques et initiateurs radicalaires utilisés dans ce procédé.**

(30) Priorité: **20.08.84 FR 8413049**

(43) Date de publication de la demande:
**26.02.86 Bulletin 86/9**

(45) Mention de la délivrance du brevet:
**25.01.89 Bulletin 89/4**

(84) Etats contractants désignés:
**AT BE CH DE FR GB IT LI LU NL SE**

(56) Documents cité:
**EP-A-0 013 474**
**EP-A-0 172 596**
**EP-A-0 173 390**

**CHEMICAL ABSTRACTS, vol. 68, no. 23, 3 juin 1968, page 10065, no. 104400a, Columbus, Ohio, US; KIYONORI SHINODA et al.: "Chlorinolysis of hexachlorobutadiene, octachlorobutene and decachlorobutane" & KOGYO KAGAKU ZASSHI 70(12), 2268-71 (1967)**

(73) Titulaire: **SOLVAY & Cie (Société Anonyme), Rue du Prince Albert, 33, B-1050 Bruxelles (BE)**

(72) Inventeur: **Franklin, James, Rue Edouard Olivier 28, B-1170 Bruxelles (BE)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

**Description**

La présente invention concerne un procédé de préparation de dérivés chlorés des composés pyridiniques tels que la 2-chloropyridine et la 2-chloro-5-trichlorométhylpyridine à partir des composés pyridiniques correspondants, par chloration substitutive en présence d'initiateurs radicalaires.

A ce jour, différentes techniques ont été développées pour la préparation de dérivés chlorés des composés pyridiniques. Une de ces techniques consiste plus particulièrement dans la préparation de la 2-chloropyridine par une réaction de chloration de la pyridine au moyen de chlore moléculaire. Cette technique a fait l'objet de mises au point de procédés pouvant être répartis en deux grandes classes.

Une première classe comprend les procédés dits thermiques, effectués en phase vapeur à haute température, généralement superieure à 250°C, voire même 300°C à 400°C et décrits notamment dans les brevets Etats-Unis 2 820 791 et 3 153 044 au nom de OLIN MATHIESON. Ces procédés présentent divers désavantages tels qu'une formation importante de goudrons, ce qui engendre des bouchages du réacteur ou des conduites, et rend dès lors difficile l'exécution du procédé en continu. En outre, ces procédés s'accompagnent d'après les inventeurs de risques d'explosion ainsi que de corrosion élevés.

Une deuxième classe de procédés comprend les procédés initiés au moyen de rayonnements lumineux ou ultraviolets. De tels procédés ont été notamment décrits dans les brevets Etats-Unis 3 297 556 au nom de OLIN MATHIETSU et 4 054 499 au nom de SEITETSU KAGAKU Co. Ces procédés, bien que permettant d'opérer à des températures plus basses que les procédés dits thermiques, présentent l'inconvénient de conduire à la formation de sous-produits goudronneux qui sa lissent les tubes lumineux et donnent lieu à une diminution subséquente du rayonnement d'initiation et dès lors du rendement de la réaction. En outre ces procédés nécessitent d'opérer en réacteurs perméables aux rayonnements d'initiation, c'est-à-dire le plus généralement en verre, ce qui rend inexploitables des réactions réalisées à pressions élevées.

La présente invention a pour but de remédier aux inconvénients des procédés connus tout en permettant de valoriser un sous-produit gênant de certaines fabrications de produits chlorés organiques. En fait, l'invention préconise d'effectuer la chloration substitutive de composés organiques au moyen de chlore moléculaire en présence d'un initiateur nouveau qui permet d'opérer à des températures modérées, en obtenant malgré tout un excellent taux de chloration. Par ailleurs l'invention permet d'opérer à des pressions égales, supérieures ou inférieures à la pression atmosphérique. L'initiateur consiste en un dérivé chloré de l'hexachlorobutadiène-1,3 qui constitue - comme on le sait - un sous-produit toxique et pratiquement dépourvu de débouchés, qui devait jusqu'ici être détruit par brûlage.

Ainsi sont connues par le document chemical Abstracts 68, 1968, 104 400a, la chloration de l'hexachlorobutadiène en $CCl_4$, $CCl_2$ $CCl_2$ et $C_2Cl_6$ à 550°C et la pyrolyse du décachlorobutane et de l'octachlorobutène en fragments $C_2$, ceux-ci étant considérés comme intermédiaires de la réaction.

L'invention concerne à cet effet un procédé pour la préparation de dérivés chlorés de composés pyridiniques par une réaction de chloration substitutive des composés pyridiniques correspondants au moyen de chlore moléculaire en présence d'un produit chloré résultant de la chloration additive de l'hexachlorobutadiène - 1,3 et servant d'initateur radicalaire, caractérisé en ce que l'initiateur consiste en un produit chloré tel que le décachlorobutane ou un octachlorobutène tel que l'octachlorobutène-1 ou un mélange contenant ces deux produits.

L'invention porte dès lors également sur l'utilisation des initiateurs de chloration substitutive des composés pyridiniques, ces initiateurs consistant en un produit chloré unique ou un mélange de produits chlorés qui résulte de la chloration additive d'hexachlorobutadiène-1,3 et qui comprend principalement du décachlorobutane et/ou un octachlorobutène tel que l'octachlorobutène-1 suivant le degré auquel la chloration additive a été poussée.

Lesdits produits chlorés obtenus par chloration additive d'hexachlorobutadiène-1,3 peuvent être préparés de manière connue en soi, par exemple par photochloration ou par chloration en phase liquide catalysée par le fer.

Ces chlorations conduisent à la formation de mélanges bruts qui contiennent en plus des produits principaux mentionnés plus haut, un peu d'hexachloréthane et éventuellement de l'hexachlorobutadiène-1,3 non transformé, ainsi qu'une faible proportion d'autres composés.

Ainsi, à titre d'exemple, on donne ci-après la composition de mélanges bruts provenant de la photochloration de l'hexachlorobutadiène-1,3, à 3°C, en g/kg:

| | Mélange A | Mélange B |
|---|---|---|
| Octachlorobutène-1 | 415 | - |
| Décachlorobutane | 303 | 812 |
| Hexachloréthane | 183 | 147 |
| Hexachlorobutadiène-1,3 | 77 | <0,5 |
| Autres composés, non identifiés | 22 | 41 |

D'autre part, un mélange brut obtenu par chloration d'hexachlorobutadiène-1,3 (1190 g de $C_4Cl_6$ et 652 g de $Cl_2$) en autoclave, à 125°C, sous une pression de 9 bars, en présence d'environ 0,1 % en poids de $FeCl_3$ (catalyseur), le produit de la chloration ayant été lavé à l'acide chlorhydrique pour éliminer le $FeCl_3$, avait la composition suivante:

2

| | Mélange C g/kg |
|---|---|
| Octachlorobutène-1 | 200 |
| Décachlorobutane | 615 |
| Hexachloréthane | 128 |
| Hexachlorobutadiène-1,1 | 25 |
| Autres composés, non identifiés | 32 |

La quantité d'initiateur mis en oeuvre dans le procédé de l'invention est comprise en général entre 0,01 et 10 moles % de la quantité totale des réactifs et diluant utilisés. Généralement cette quantité est de 0,01 à 5 moles % et de préférence on travaille avec 0,1 à 2 moles % d'initiateur par rapport aux réactifs et diluants mis en oeuvre.

Le chlore moléculaire et les composés pyridiniques sont habituellement mis en oeuvre dans des rapports molaires compris entre 0,1 et 20 moles de chlore par mole de composé pyridinique. Ce rapport dépend notamment du nombre d'atomes d'hydrogène que l'on désire substituer. De préférence ce rapport varie entre 0,2 et 15 moles et tout particulièrement préférés sont des rapports molaires situés entre 0,3 et 10 moles de chlore par mole de pyridine. Dans le cas de la fabrication de la 2-chloropyridine à partir de la pyridine le rapport préféré est compris entre 0,2 et 2 moles de chlore par mole de pyridine. Dans le cas de la fabrication de la 2-chloro-5-trichlorométhylpyridine le rapport préféré est compris entre 2 et 20 moles de chlore par mole de 3-méthylpyridine (β-picoline) mis en oeuvre.

Les initiateurs de chloration susdits conviennent pour les réactions en phase gazeuse ou en phase liquide. Ils peuvent être utilisés à la pression atmosphérique ou à une pression plus élevée. La température réactionnelle dépend bien entendu de la nature du composé à chlorer et des autres conditions opératoires, mais de toute manière:

- à taux de chloration et temps de séjour donnés, l'emploi des initiateurs selon l'invention permet de réduire la température et d'améliorer la sélectivité et de réduire la formation de goudrons et l'apparition de salissage observé à haute température;

- à température et temps de séjour donnés, on peut atteindre un taux de chloration plus élevé, ce qui peut permettre une économie d'énergie par réduction de la quantité de composés pyridiniques à recycler;

- à température et taux de chloration donnés, on peut réduire le temps de séjour, ce qui revient à augmenter la productivité du réacteur.

Outre les réactifs et initiateurs déjà cités on peut avantageusement mettre en oeuvre dans le procédé selon l'invention des additifs tels que de la vapeur d'eau, de l'azote et/ou d'autres gaz ne participant pas à la réaction de chloration proprement dite. Habituellement on réalise la réaction de chloration en présence de vapeur d'eau à raison de 0,1 à 25 moles par mole de composé pyridinique mis en oeuvre. De préférence on utilise des quantités de vapeur d'eau de 1 à 15 moles par mole de composé pyridinique. L'adjonction de ces additifs et plus particulièrement de la vapeur d'eau peut être réalisée de toutes les façons. Une façon avantageuse consiste à mélanger préalablement l'eau et le composé pyridinique, à envoyer ce mélange dans un évaporateur et à injecter ensuite les vapeurs ainsi obtenues dans le réacteur de chloration proprement dit.

On a également constaté qu'il peut être souhaitable dans certains cas afin de minimiser la surchauffe du mélange réactionnel et afin d'éviter la condensation de produits de réaction peu volatils d'exécuter la réaction de chloration en présence d'additifs qui agissent comme diluants mais qui sont inertes vis-à-vis des réactifs et des initiateurs intervenant dans la réaction de chloration. Comme additifs on utilise de préférence des dérivés chlorés de composés aliphatiques tel que le tétrachlorure de carbone ou des produits inorganiques tels que le chlorure d'hydrogène ou l'azote.

De préférence on opère avec le tétrachlorure de carbone.

En général les additifs organiques halogénés sont ajoutés au milieu réactionnel à raison de 1 à 25 moles par mole de composé pyridinique mis en oeuvre. Lorsque l'on utilise du tétrachlorure de carbone, comme additif organique, les quantités préférées se situent entre 1,5 et 15 moles par mole de composé pyridinique mis en oeuvre.

Le procédé selon l'invention peut être réalisé dans tout appareillage ou tout réacteur permettant de réunir les conditions opératoires décrites ci-avant. De bons résultats ont été obtenus dans l'appareillage décrit aux exemples ci-après.

Les dérivés chlorés des composés pyridiniques obtenus selon le procédé de l'invention peuvent être utilisés dans toutes les applications connues de ce produit c'est-à-dire comme intermédiaire chimique pour la fabrication notamment de produits destinés à l'agriculture, de cosmétiques et de produits pharmaceutiques.

Les exemples qui suivent servent à illustrer l'invention sans toutefois en limiter la portée.

**Exemple 1**

Dans cet exemple on a effectué la chloration substitutive de la pyridine à 230°C en vue d'obtenir la 2-chloropyridine.

La chloration de la pyridine en phase gazeuse a été effectuée à la pression atmosphérique dans un réacteur mélangeur continu sphérique d'environ 1 dm$^3$, en pyrex, auto-agité par jets gazeux (Chem. Eng. Sci., 1973, 28, p. 129 - 137), les réactifs étant introduits sous forme gazeuse à l'aide d'un injecteur à quatre tuyères placé au milieu de la sphère.

Le réacteur est placé dans une enceinte à l'intérieur de laquelle l'air est chauffé électriquement et brassé à l'aide d'une turbine, afin de maintenir la température réactionnelle désirée. Le mélange pyridine-eau est alimenté via un évaporateur tubulaire vertical, chauffé électriquement. Le diluant (CCl$_4$), destiné à modérer l'effet thermique de la réaction est alimenté via un deuxième évaporateur identique. Le chlore gazeux est injecté dans le pied de l'évaporateur à CCl$_4$. L'initiateur, dans le cas où on l'a utilisé, est ajouté sous forme liquide, en solution dans du CCl$_4$, par une dérivation donnant dans le conduit d'introduction du mélange gazeux CCl$_4$ + Cl$_2$. Les produits de chloration sortent du réacteur par une tubulure diamétralement opposée à l'introduction, puis ils sont condensés, traités par du NaOH aqueux pour neutraliser le chlore résiduaire et le HCl formé. Après décantation, la phase organique est séparée de la phase aqueuse; celle-ci est soumise à une extraction au moyen de chloroforme et la phase organique ainsi que l'extrait chloroformique sont analysés par chromatographie en phase vapeur. L'azote total dans la phase aqueuse extraite est déterminé par la méthode de Kjeldahl. Les conditions et les résultats des essais sont donnés au tableau I ci-après.

## TABLEAU 1

| | | ESSAI DE REFERENCE, SANS INITIATEUR | ESSAI AVEC INITIATEUR (MELANGE C) |
|---|---|---|---|
| CONDITIONS | | | |
| - température dans le réacteur | °C | 230 | 229 |
| - température sortie évaporateur I (CCl$_4$ + Cl$_2$) | °C | 164 | 163 |
| - température sortie évaporateur II (pyridine + H$_2$O) | °C | 182 | 188 |
| - introduction de l'initiateur | | néant | solution à 56 % en poids dans CCl$_4$ |
| - temps de séjour moyen* | s | 10 | 10 |
| - rapports molaires des réactifs | | | |
| pyridine | mol/mol | 1 | 1 |
| Cl$_2$ | mol/mol | 0,67 | 0,68 |
| CCl$_4$ (diluant inerte) | mol/mol | 2,4 | 2,4 |
| H$_2$O (diluant inerte) | mol/mol | 1,1 | 1,1 |
| initiateur (somme de ses constituants) | mol/mol | 0 | 0,06 |
| - teneur du mélange réactionnel en initiateur | % mol | 0 | 1,1 |

$$\text{* temps de séjour} = \frac{\text{volume du réacteur}}{\text{débit volumique des réactifs à la température réactionnelle}}$$

| | | | |
|---|---|---|---|
| RESULTATS | | | |
| - répartition des produits de chloration | | | |
| pyridine | % mol | 86,2 | 52,0 |
| 2-chloropyridine | % mol | 7,2 | 42,6 |
| 3- et 4-chloropyridines | % mol | 0,1 | 0,3 |
| 2,6-dichloropyridine | % mol | 0,1 | 4,3 |
| autres dichloropyridines | % mol | < 0,1 | 0,8 |
| composés azotés non extractibles (par CHCl$_3$) dans la phase aqueuse | % mol | 6,3 | < 0,1 |
| - 2-chloropyridine formée/Cl$_2$ mis en oeuvre | mol/mol | 0,11 | 0,63 |
| - taux de chloration 1 (moles Cl fixé sur pyridine/moles de pyridine mise en oeuvre) | mol/mol | 0,08 | 0,53 |
| - taux de chloration 2 (moles Cl fixé sur pyridine/moles de Cl$_2$ mis en oeuvre) | mol/mol | 0,11 | 0,78 |

Ce tableau montre qu'en l'absence d'initiateur, à 230°C, on n'observe qu'une faible conversion de la pyridine en 2-chloropyridine; il se forme une quantité de sous-produits hydrosolubles (non extractibles au chloroforme) presque aussi importante que la quantité de 2-chloropyridine produite.

L'addition de 1,1 % mol de produit de la chloration additive de l'hexachlorobutadiène-1,3 permet d'accroître très sensiblement l'avancement de la réaction:
- le rapport 2-chloropyridine formée/$Cl_2$ mis en oeuvre passe de 0,11 à 0,63 mol/mol;
- le rapport Cl fixé sur pyridine/pyridine mise en oeuvre passe de 0,08 à 0,53 mol/mol;
- le rapport Cl fixé sur pyridine/$Cl_2$ mis en oeuvre passe de 0,11 à 0,78 mol/mol.

**Exemple 2**

On a réalisé la chloration substitutive de la β-picoline en vue d'obtenir la 2-chloro-5-trichlorométhylpyridine. L'installation et la méthode utilisées étaient identiques à celles de l'exemple 1. Les conditions et les résultats des essais sont donnés au tableau 2 ci-après.

On peut y voir qu'en absence d'initiateur (essai 1), vers 300°C, il se produit une certaine chloration de la β-picoline, mais le rendement en 2-chloro-5-trichlorométhylpyridine est faible (env. 2 % de la β-picoline mise en oeuvre), les produits principaux étant des β-picolines mono-, di- et tri-chlorées.

L'addition de 0,3 % mol de produit de la chloration additive de l'hexachlorobutadiène-1,3 (essai 2) permet de porter le rendement en 2-chloro-5-trichlorométhlylpyridien de 2 à 27 % par rapport à la β-picoline mise en oeuvre. Le taux de chloration (moles de Cl fixé sur la β-picoline/moles de β-picoline mise en oeuvre) passe de 1,70 à 3,22 mol/mol. La proportion de β-picoline transformée en sous-produits hydrosolubles (non extractibles au chloroforme) diminue de 6,1 à < 0,6 % mol de la β-picoline mise en oeuvre.

Les essais 3 et 4 réalisés respectivement avec 1,50 et 1,53 % mol de produit de chloration de l'hexachlorobutadiène-1,3 permettent de confirmer les résultats de l'essai 2.

**TABLEAU 2**

| | | | ESSAI 1 DE REFRENCE, SANS INITIATEUR | ESSAI 2 AVEC INITIATEUR (MELANGE B) | ESSAI 3 AVEC INITIATEUR (MELANGE C) | ESSAI 4 AVEC INITIATEUR (MELANGE C) |
|---|---|---|---|---|---|---|
| CONDITIONS | | | | | | |
| - température dans le réacteur | °C | | 301 | 303 | 301 | 250 |
| - température sortie évaporateur I ($CCl_4 + Cl_2$) | °C | | 136 | 137 | 143 | 104 |
| - température sortie évaporateur II (β-picoline + $H_2O$) | °C | | 185 | 192 | 144 | 135 |
| - introduction de l'initiateur | | | néant | solution à 11 % poids dans $CCl_4$ | solution à 66 % poids dans $CCl_4$ | solution à 47 % poids dans $CCl_4$ |
| - temps de séjour moyen | s | | 10 | 10 | 20 | 20 |
| - rapports molaires des réactifs β-picoline | mol/mo | | 1 | 1 | 1 | 1 |
| $Cl_2$ | mol/mol | | 6,3 | 6,8 | 6,2 | 6,0 |
| $CCl_4$ (diluant inerte) | mol/mol | | 11 | 11 | 2,1 | 2,2 |
| $H_2O$ (diluant inerte) | mol/mol | | 1,1 | 1,1 | 1,2 | 1,1 |
| initiateur (somme des constituants) | mol/mol | | 0 | 0,06 | 0,16 | 0,16 |
| - teneur de mélange réactionnel en initiateur | % mol | | 0 | 0,30 | 1,50 | 1,53 |

RESULTAT
- répartition des produits de
chloration

| | | | | | |
|---|---|---|---|---|---|
| β-picoline | % mol | 15,2 | 0,9 | 0,1 | 2,6 |
| monochloro-β-picolines | % mol | 20,7 | 2,9 | 0,5 | 4,0 |
| dichloro-β-picolines | % mol | 29,3 | 11,6 | 1,6 | 9,6 |
| trichloro-β-picolines | % mol | 24,2 | 43,7 | 25,7 | 43,5 |
| 2-chloro-5-trichlorométhyl-pyridine | % mol | 2,1 | 26,5 | 44,1 | 25,5 |
| 2-chloro-3-trichlorométhylpyridine | % mol | 0,6 | 8,8 | 12,6 | 8,1 |
| autres tetrachloro-β-picolines | % mol | 1,8 | 1,9 | 2,5 | 2,5 |
| 2,6-dichloro-3-trichlorométhyl-pyridine | % mol | < 0,1 | 3,2 | 11,0 | 2,3 |
| composés azotés non extractibles dans la phase aqueuse | % mol | 6,1 | < 0,6 | 1,8 | 1,9 |
| - 2-chloro-5-trichlorométhyl-pyridine formée/β-picoline mise en oeuvre | mol/mol | 0,02 | 0,27 | 0,44 | 0,26 |
| - taux de chloration (moles de Cl fixé sur la β-picoline/moles de β-picoline mise en oeuvre) | mol/mol | 1,70 | 3,22 | 3,73 | 3,10 |

**Revendications**

1. Procédé pour la préparation de dérivés chlorés de composés pyridiniques par une réaction de chloration substitutive des composés pyridiniques correspondants au moyen de chlore moléculaire en présence d'un produit chloré résultant de la chloration additivé de l'hexachlorobutadiène - 1,3 et servant d'initiateur radicalaire caractérisé en ce que l'initiateur consiste en un produit chloré tel que le décachlorobutane ou un octachlorobutène tel que l'octachlorobutène-1 ou un mélange contenant ces deux produits.

2. Procédé suivant la revendication 1 caractérisé en ce qu on utilise l'initiateur en une quantité comprise entre 0,01 et 5 mol % par rapport aux réactifs et aux diluants mis en oeuvre.

3. Procédé suivant l'une des revendications 1 et 2 caractérisé en ce qu'on opère à une pression supérieure à la pression atmosphérique.

4. Procédé suivant l'une des revendications 1 à 3 caractérisé en ce qu'il est appliqué à la chloration substitutive de la pyridine en 2-chloropyridine.

5. Procédé suivant l'une des revendications 1 à 3 caractérisé en ce qu'il est appliqué à la chloration substitutive de la β-picoline en 2-chloro-5-trichloraméthylpyridine.

6. Utilisation comme initiateur d'un produit chloré ou d'un mélange de produits chlorés qui résulte de la chloration additive d'hexachlorobutadiène-1,3, comprenant principalement du décachlorobutane et/ou un octachlorobutène tel que l'octachlorobutène-1, pour l'initiation de réactions de chloration substitutive de composés pyridiniques.

7. Utilisation suivant la revendication 6 caractérisée en ce què l'initiateur est obtenu par photochloration d'hexachlorobutadiène-1,3.

8. Utilisation suivant la revendication 6 caractérisée en ce que l'initiateur est obtenu par chloration d'hexachlorobutadiène-1,3 en phase liquide, catalysée par le fer.

**Patentansprüche**

1. Verfahren zur Herstellung von chlorierten Derivaten von Pyridinverbindungen durch eine substitutive Chlorierungsreaktion entsprechender Pyridinverbindungen unter Zuhilfenahme von molekularem Chlor in Gegenwart eines chlorierten Produkts, das aus der zusätzlichen Chlorierung von Hexachlorbutadien-1,3 resultiert und als radikalartiger Initiator dient, dadurch gekennzeichnet, daß der Initiator aus einem chlorierten Produkt wie dem Decachlorbutan oder einem Octachlorbuten wie dem Octachlorbuten-1 oder einer Mischung enthaltend diese zwei Produkte besteht.

2. Verfahren nach Anspruch 1, dadurch gekennzeichnet, daß man den Initiator in einer Menge zwischen 0.01 und 5 Mol % bezogen auf eingesetzte Reagenzien und Verdünnungsmittel verwendet.

3. Verfahren nach einem der Ansprüche 1 und 2, dadurch gekennzeichnet, daß man bei einem höheren Druck als dem atmosphärischen Druck arbeitet.

4. Verfahren nach einem der Ansprüche 1 - 3, dadurch gekennzeichnet, daß es bei der substitutiven

6

Chlorierung von Pyridin zu 2-Chlorpyridin angewandt wird.

5. Verfahren nach einem der Ansprüche 1 bis 3, dadurch gekennzeichnet, daß es bei der substitutiven Chlorierung von β-Picolin zu 2-Chlorstrichlormethylpyridin angewandt wird.

6. Verwendung als Initiator eines chlorierten Produkts oder einer Mischung von chlorierten Produkten, das aus der zusätzlichen Chlorierung von Hexachlorbutadien-1,3 resultiert, enthaltend hauptsächlich Decachlorbutan und/oder ein Octachlorbuten wie das Octachlorbuten-1 zur Initiation von substitutiven Chlorierungsreaktionen von Pyridinverbindungen.

7. Verwendung nach Anspruch 6, dadurch gekennzeichnet, daß der Initiator durch Photochlorierung von Hexachlorbutadien-1,3 erhalten wird.

8. Verwendung nach Anspruch 6, dadurch gekennzeichnet, daß der Initiator durch Chlorierung von Hexachlorbutadien-1,3 in flüssiger Phase, katalysiert durch Eisen, erhalten wird.

**Claims**

1. Process for preparing chlorinated derivatives of pyridine compounds by a substitutive chlorination reaction of the corresponding pyridine compounds, using molecular chlorine in the presence of a chlorinated product which results from the additive chlorination of hexachloro-1,3-butadiene and which serves as a free-radical initiator, characterized in that the initiator consists of a chlorinated product such as decachlorobutane or an octachlorobutene such as octachloro-1-butene or a mixture containing these two products.

2. Process according to Claim 1, characterised in that the initiator is used in an amount between 0.01 and 5 mol % relative to the reagents and diluents employed.

3. Process according to Claims 1 and 2, characterised in that the reaction is performed at a pressure greater than atmospheric pressure.

4. Process according to one of Claims 1 to 3, characterised in that it is applied to the substitutive chlorination of pyridine to 2-chloropyridine.

5. Process according to one of Claims 1 to 3, characterised in that it is applied to the substitutive chlorination of β-picoline to 2-chloro-5-(trichloromethyl)-pyridine.

6. Use as initiator of a chlorinated product or a mixture of chlorinated products which results from the additive chlorination of hexachloro-1,3-butadiene, mainly comprising decachlorobutane and/or an octachlorobutene such as octachloro-1-butene, for the initiation of substitutive chlorination reactions of pyridine compounds.

7. Use according to Claim 6, characterised in that the initiator is obtained by photochlorination of hexachloro-1,3-butadiene.

8. Use according to claim 6, characterised in that the initiator is obtained by iron-catalysed chlorination of hexachloro-1,3-butadiene in the liquid phase.